# EUROPÄISCHE PATENTANMELDUNG

(11) **EP 1 048 280 A1**
(43) Veröffentlichungstag der Anmeldung: **02.11.2000**
(21) Anmeldenummer: 99108187.8
(22) Anmeldetag: 27.04.1999
(51) Int. Cl.: A61F 15/00

(54) **Wiederverwendbare Nässeschutzhülle**

(71) Anmelder: Dontenvill, Christian, 89081 Ulm (DE); Beutler, Friedhelm, 88212 Ravensburg (DE)
(72) Erfinder: Dontenvill, Christian, 89081 Ulm (DE); Beutler, Friedhelm, 88212 Ravensburg (DE)

(57) **Zusammenfassung**

Wasserdichte Schutzhülle (1), die eine einfache einhändige Selbstapplizierung beim Anund Ausziehen der Nässeschutzhülle ermöglicht, eine Mehrfachverwendung gewährleistet, aber keine Latex-Allergiegefahr verbirgt. Die physiologisch verträgliche Nässeschutzhülle (1) besteht zumindest im Bereich der Öffnung (2) aus einer Folie die beim Zusammenraffen auf der Haut keine wasserdurchlässigen Kanäle bildet. Die Nässeschutzhülle (1) dient zum Abdecken einer Körperextremität die wegen Gipsverband oder sonstigen Verbänden beim Duschen vor Nässe zu schützen ist. Sie ist aus wasserdichter, mikrogeprägter 20 bis 30 µm dicken Folie und einer die Extremität wasserdicht auf der Haut umschließenden Öffnung (2). Die Nässeschutzhülle kann aus PE, PP, PVC, oder LD-PE Folie bestehen, insbesondere überwiegend aus LLD-PE. An der Hüllenöffnung (2) ist ein Gummiband (5) eingesäumt oder ein Spanngurt angeordnet.

## Beschreibung

Die vorliegende Erfindung betrifft eine wasserdichte Schutzhülle.

Für den oben genannte Anwendungsbereich sind Duschschutzhüllen bekannt. Problematisch war jedoch stets die Abdichtung zwischen der Hüllenöffnung und dem Körperteil.
DE 195 31 856 A1 betrifft zwar keine Nässeschutzhülle, sondern einen Folienanzug für Voll- und Sitzbäder, in den Badeflüssigkeit eingegossen werden kann. Der Sack besteht aus flexibler Folie, die mit einer Schnur zugezogen wird, die außen oder in einem Tunnel liegt. In dem Tunnel kann auch ein Gummiband eingelegt sein. Das Folienmaterial liegt nicht flach an der Haut und wird deshalb beim Abdichten mit der Schnur oder dem Gummizug gerafft. Die Folie schiebt sich dabei wie ein Akkordäon zusammen und ist deshalb auf der Haut kein wasserdichter Abschluß. Nässeschutzhüllen sollen aber an der Öffnung dicht mit der Haut abschließen.
EP 0183 992 A1 erkennt das Problem, daß eine Abdichtung nicht erfolgt, wenn Sackartige Kunststoffhüllen mit Schnürren, Gummiringen oder Bändern am Körperteil befestigt werden. Deshalb wird in diesem Dokument ein klebender, bandförmiger Dichtstreifen vorgeschlagen, obwohl in diesem Dokument bereits erwähnt ist, daß Klebestreifen im allgemeinen nicht wasserdicht sind, schlecht an feuchten Körperstellen kleben und nur unter Schmerzen zu entfernen sind, wenn sie an Haaren kleben. Weiterhin besteht bei einem auf der Haut befestigten Klebestreifen eine Allergiegefahr, insbesondere die Gefahr einer Latexallergie bei Klebstoff auf Kautschukbasis. Darüber hinaus ist das Anbringen des Klebestreifens kaum ohne fremde Hilfe möglich, denn der Klebestreifen muß um den gesamten Umfang entlang hälftig die Folie erfassen und mit der anderen Hälfte auf der Haut kleben und dabei glatt anliegen und dennoch den weiten Umfang der Hüllenöffnung auf den kleineren Umfang der Körperextremität reduzieren.
Die DE 41 26 139 A1 betrifft eine Schutzabdeckung, die durch ihre Eigenspannung auf der Haut abschließt. Damit steht man aber vor dem Dilemma, daß entweder die Dichtigkeit nicht gewährleistet wird oder die Abdeckung nicht ohne Druckbelastung über den Verband gezogen werden kann. Das Material ist teuer und eine individuelle Anpassung kaum möglich. Alternativ werden auch Reiß-, Klett-, Klebe- oder Bindeverschlüsse vorgeschlagen. Das Einarbeiten eines Reiß- oder Klettverschlusses ist teuer. Bindeverschlüsse sind undicht und einschnürend.
Nach DE 89 11 168 U1 läßt sich ein Schutzüberzug für einen Verband mit einem elastischen Bund abdichten und zwar vorzugsweise durch die Elastizität eines elastische anliegenden Gummischlauchs, oder eines Gummizugs sowie einer festziehbaren Kordel. Ungelöst bleibt auch nach diesem Dokument sowohl das Dilemma mit der Eigenelastizität des Schlauchs, als auch das Eindringen von Wasser aufgrund des Akkordäon-Effekts.
Nach DE 297 16 769 U1 wird ein dehnbares elastisches Band so angebracht, daß es bei Gebrauch direkten Kontakt zur Haut hat, d.h. die Gefahr einer Latexallergie ist gegeben.
DE 92 12 449 U1 schlägt ein Wickelband zum Abdichten vor. Diese Ausführung ist umständlich für den Patienten anzulegen und darüber hinaus sehr aufwendig und teuer. Gemäß DE 92 10 402 U1 eignet sich zur Abdichtung Schaumstoff oder ein gefüllter Gummischlauch. Die an sich geniale Idee scheitert in der Praxis daran, daß die verknüpfung mit der Hülle technisch sehr aufwendig oder nur mit teuerem Material realisierbar ist. Deshalb ist der Artikel letztlich unwirtschaftlich.
In DE 92 05 982 U1 ist eine Wickelfolie vorgeschlagen. Gleichgültig, ob die Fixierungswickelbänder angenäht oder von außen angebracht sind, liegt das Folienmaterial selbst bei festem abschnürendem Festziehen nicht flach und somit nicht dicht auf der Haut.
Das Bedürfnis nach einem praktischen und preiswerten aber dennoch wasserdichten Verband- und Gipsschutz konnte trotz dieser und vieler weiteren Bemühungen bislang nicht erfüllt werden.

Die Aufgabe der vorliegenden Erfindung besteht im Bereitstellen einer Nässeschutzhülle, die eine einfache einhändige Selbstapplizierung beim An- und Ausziehen der Nässeschutzhülle ermöglicht, eine Mehrfachverwendung gewährleistet, aber keine Latex-Allergiegefahr verbirgt.

Erfindungsgemäß wird eine physiologisch verträgliche Nässeschutzhülle bereitgestellt, die zumindest im Bereich der Öffnung aus einer Folie besteht, die beim Zusammenraffen auf der Haut keine wasserdurchlässigen Kanäle bildet. Folie die mit der Haut in Berührung kommen kann, ist physiologisch verträglich. Die Hülle kann einerseits aus einer einheitlichen Folie hergestellt werden, die keine wasserdurchlässigen Kanäle beim Zusammenraffen bildet. Anderseits kann die Hülle ein Verbundmaterial sein das lediglich (oder zumindest) an der Hüllenöffnung einen Bereich aufweist, der beim Zusammenraffen keine wasserdurchlässigen Kanäle bildet. Es reicht aus, einen LLD-PE (Linear-low-Density-Polyäthylen)-Streifen mit Schweißnähten auf einer LD-PE Folie zu befestigen, ins besondere dabei ein Gummiband einzuschließen. Die Nässeschutzhülle besteht aus einer wasserdichten Folie und ist latexfrei. Der Erfindungsgemäße Nässeschutz kann im Klinikbereich, auf Unfallstationen, in der Rehabilitation sowie im Privatbereich und insbesondere in der häuslichen Pflege angewendet werden.
Diese Hilfe ist unter anderem für alleinstehende Personen und Behinderte sehr praktisch. Besonders geeignet ist eine ungefähr 25 µm dicke Hülle aus mikrogeprägter LD-PE Folie.
Mikrogeprägte Folien lassen sich durch Kalandrieren herstellen. Die Mikroprägung verleiht der Folie eine verbesserte Reißfestigkeit. Deshalb erlaubt die Mikroprägung den Einsatz dünnerer Folien verbunden mit einer entsprechenden Materialersparnis. Die Folie kann sowohl transparent als auch eingefärbt sein. Die Hülle kann mit einem Spanngurt auf der Haut abgedichtet werden. Der Spanngurt kann lose um die Hülle angezogen werden. Vorzugsweise ist er jedoch an der Hülle befestigt, um das Anbringen des Nässeschutzes zu erleichtern.
In weiterer vorteilhafter Ausführung ist die Hülle am Rand gesäumt und im Saum ein Gummiband eingeschlossen.

Nach erfolgten Untersuchungen, die nachstehend dokumentiert sind, wird erfindungsgemäß eine wiederverwendbare Nässeschutzhülle bereitgestellt, die am Öffnungsrand gesäumt ist, deren Saum wenigstens eine Schweißnaht aufweist und im Saum wenigstens ein Kautschukband eingeschweißt ist. Diese Nässeschutzhülle bietet einen zuverlässigen Nässeschutz ohne Akkordäon-Effekt und wird bei Hautverletzungen, chirurgischen Eingriffen, Gips- und sonstigen Verbänden an den Armen, Beinen sowie am Kopf und Ohren eingesetzt, ohne den Patienten in irgendeinerweise einzuschnüren.
Da angenommen werden kann, daß nicht alle Personen gleiche Körper-Konstitutionen haben, (ältere Personen oder Jugendliche sind allgemein etwas dünner an Ihren Armen oder Beinen) wurde von einem Minimalumfang der Extremität von 240 mm ausgegangen den es galt garantiert abzudichten. Es sollte überhaupt kein Wasser eindringen, den Verband oder Gips nässen oder sogar aufweichen. Damit nicht okkulte Flüssigkeit übersehen wird, wurde das getestete Segment mit Papier eingewickelt und mit Klebefilm an der Haut festgeklebt um ein Verrutschen zu vermeiden. Ziel der Tests war es einen Duschvorgang unter extremen Bedingungen zu simulieren. Jeweils fünf Minuten lang wurde ein starker Duschbrausen-Strahl aus ca. 30 cm Entfernung auf den Rand der Duschhülle gerichtet. Der komplette Umfang des Schutzes wurde so mit direktem Wasserdruck getestet. Anfangsversuche mit normaler, handelsüblicher 50 µm starken PE-Folie zeigten , daß eine Dichtigkeit an einer dünnen Extremität z.B. Arm nicht so einfach zu erreichen war. Die Annahme, daß ein eingeschweißtes Gummiband mit 5 mm breite ausreichend wäre, erwies sich schnell als falsch. Eine schrittweise Vergrösserung der Gummibandbreite von 6mm auf 7mm, 8mm etc. und Verkleinerung des Gummiband-Durchmessers (halbe Gummiband-Länge) von 70 mm bis auf 50 mm zeigte immer wieder, daß Wasser eindrang. Die so erfolgten Tests wurden bis zu einer Gummibandbreite von 20 mm durchgeführt. Die Ergebnisse waren nicht zufriedenstellend. Immer wieder war das als Verbandersatz umwickelte Papier durchnäßt. Obwohl der eingeschweißte Gummi die Haut immer weiter einschnürte drang Wasser unter den Nässeschutz. Die Anfangsbreite der Öffnung der Hülle wurde durch den geringeren Durchmesser des eingeschweißten Gummibandes auf ein Minimum reduziert. Die somit wie ein Akkordäon um das Gummiband geraffte Folienöffhung bildete dünne Kanäle durch die das Wasser unwillkürlich Einlaß fand.
Daraufhin wurde die Folienstärke von 50 µm schrittweise bis auf 25 µm reduziert. Die Tests wurden jeweils mit den verschiedenen Gummibreiten wiederholt. Sogar bis zu einer maximalen erträglichen Abschnürungsgrenze mit einem Gummiband in 15mm Breite waren die Testsergebnisse nicht zufriedenstellend. Es waren immer noch Kanäle zu erkennen. Die Folie war nicht dicht. Weitere Herabsetzung der Folienstärke wurde nicht erprobt, da die Reißfestigkeit bei zu dünner Folie nicht mehr gewährleistet ist. Schon beim anziehen der Duschhülle und leichtem Ziehen würde die Folie reißen. Ein Benutzen oder Wiederbenutzen der Hülle wäre ausgeschlossen. Die PE-Folie war für dieses Vorhaben nicht geeignet. Eine weitere Verkleinerung des Gummiband-Durchmessers kam wegen Einschnürungen nicht in Frage. Eine Vergrößerung der Gummiband-Breite verhinderte nicht, daß sich trotzdem Kanäle bilden.

Bei weiteren Tests ergaben sich überraschend, daß leicht geprägte Folie aus 25 µm starker LLDPE Folie keine wasserdurchlässigen Kanäle bildet. Kalandrierte LLD-PE-Folie ist weich und trotzdem reißfest. Das Kalandrieren der Folie erhöht die Reißfestigkeit des Materials. Es ist bekannt, daß flächige Materialien mit Falten und ähnlichen Strukturen versteift werden und die Belastbarkeit erhöht wird. Auf dem gleichen Prinzip beruht die erhöhte Belastbarkeit mikrogeprägter Folie. Überraschenderweise bleibt beim Kalandrieren nicht nur der weiche anschmiegsame Charakter der Folie erhalten, sondern die dadurch erhaltene mikrogeprägte Folie bildet beim Ziehharmonika - artigen Zusammenraffen keine wasserdurchlässigen Kanäle. Um dem Ausbilden von wasserdurchlässigen Kanälen weiter vorzubeugen, soll das Zusammenziehen -aufgrund dessen sich wasserdurchlässige Kanäle bildeten- gering gehalten werden. Der Umfang der Hülle sollte deshalb nicht wesentlich größer sein als der Umfang, der vor Nässe zu schützenden Körperextremität.
Bei relativ zur Extremität gering gehaltenen Folienumfang liegt die Folie noch besser auf der Haut, ohne Kanäle zu bilden. Ein Sortiment verschiedener Größen, angefangen für Kleinkinderärmchen und endend für Beine Übergewichtiger, braucht zu mittleren Größen keine Unterschiede für Arm- und Beinschutz aufzuweisen. Der Öffnungsumfang der Hülle ist üblicherweise kleiner als die Hüllenlänge. Sind die Hüllen für Kopf oder Ohren bestimmt, ist dies aber eher umgekehrt.
Mit der vorliegenden Erfindung wird ein in wiederverwendbarer, selbstapplizierender Weise, ohne Allergiegefahr und ohne Bildung von wassedurchlässigen Kanälen, ein beim Duschen zuverlässiger Nässeschutz bei Hautverletzungen, chirurgischen Eingriffen, Gips- und sonstigen Verbänden an den Beinen, Armen sowie am Kopf ermöglicht Er verhindert Wundinfektionen durch Nässe und verkürzt die Heilungsdauer.

Eine erfindungsgemäße Nässeschutzhülle aus durch Kalander geprägtem LLD-PE (Linear Low Density Polyäthylen), ist frei von jedem Latex und kann zwischen 0,02 mm und 0,035 mm dick sein, damit sie nicht zu schnell reißt und trotzdem angenehm weich ist und ohne wasserdurchlässige Kanäle auf der Haut abdichtet.

### Beispiele:

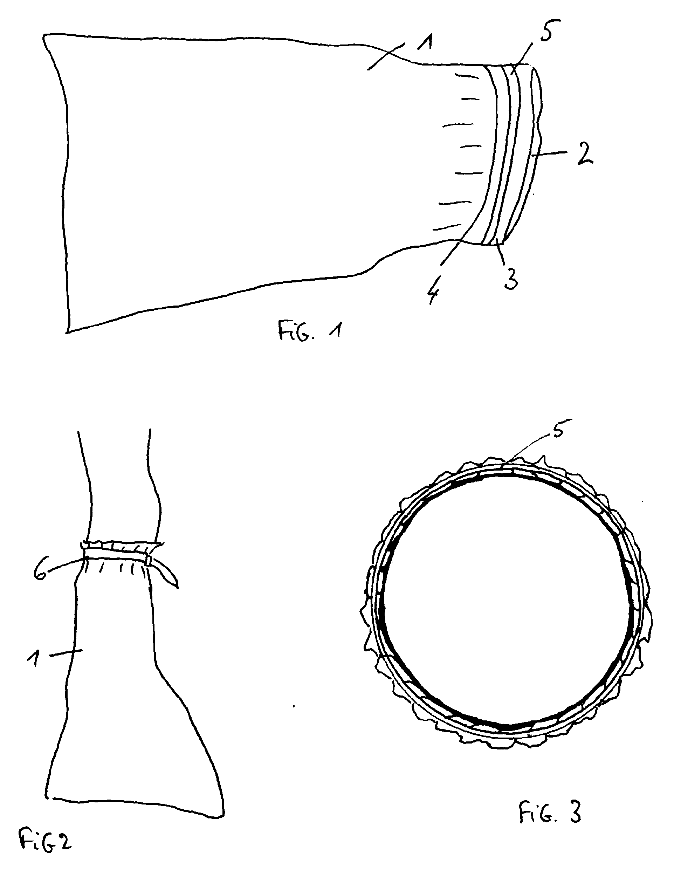

Fig. 1 zeigt die Nässeschutzhülle mit einem durch Umschlag am Rand eingeschweißten Gummizug. An deren Öffnung 2 ist die Folie 1 gesäumt. Der Saum 3 ist durch eine Schweißnaht 4 insbesondere durch eine Hochfrequenzschweißnaht 4 geschweißt. Im Saum 3 ist ein Gummizug 5 eingeschlossen. Der Gummizug 5 hat ein Verhältnis Breite zu Dicke von mindestens 3.

In einer weiteren vorteilhaften Ausführung nach Fig. 2 besteht die Hülle 1 aus einer sackförmig ausgebildeten Folie, die weder Saum 3 noch darin freiliegenden Gummizug 5 aufweist, sondern mit einem separatem Besfestigungsmittel 6 wie Klett-, Klebe- oder Druckknopfband abgedichtet wird.

Fig. 3 zeigt im Schnitt die ohne Akkordäon- Effekt flach auf der Haut anliegende Folie 1, die auf der Haut mittels eingeschweißtem Gummizug 5 oder separat aufgelegten oder außen aufgebrachten Befestigungsmittel 6 ohne übriggebliebene Kanäle abdichtet. Mit diesem Gummiband 5 schmiegt sich die kalandrierte Folie 1 an der Hüllenöffnung 2 zwischen Gummi 5 und Haut ohne wasserdurchlässige Kanäle zu bilden. Es dringt kein Wasser ein. Das umwickelte Testpapier wies keine Nässespuren auf. Das Zusammenwirken der anschmiegsamen Folie mit dem eingeschweißten, angepaßten, nicht abschnürenden Gummiband ermöglicht hohen Tragekomfort und die seit langer Zeit erwünschte Dichtigkeit als Nässeschutzhülle.
Eine Hülle mit einem Umfang von 32 cm und einem eingeschweißten Gummiband von 13 mm Breite, 1 mm Stärke und 50 mm Durchmesser wird über einen Unterarm mit ca. 24 cm Umfang gezogen.

## Patentansprüche

1. Nässeschutzhülle zum Abdecken einer Körperextremität, aus wasserdichter Folie und einer die Extremität wasserdicht auf der Haut umschließenden Öffnung, dadurch gekennzeichnet, daß
die Folie physiologisch verträglich ist und beim Zusammenraffen auf der Haut keine wasserdurchlässigen Kanäle bildet.

2. Nässeschutzhülle nach Anspruch 1, dadurch gekennzeichnet, daß die Folie mikrogeprägt ist.

3. Nässeschutzhülle nach Anspruch 1 und 2, dadurch gekennzeichnet, daß die Folie 20 bis 35, insbesondere 25 bis 30 µm dick ist

4. Nässeschutzhülle nach einem der Ansprüche 1 bis 3, dadurch gekennzeichnet, daß die Folie aus PE, PP, PVC, oder LD-PE besteht, insbesondere überwiegend aus LLD-PE.

5. Nässeschutzhülle nach einem der Ansprüche 1 bis 4, dadurch gekennzeichnet, daß an der Hüllenöffnung ein Gummiband eingesäumt ist.

6. Nässeschutzhülle nach Anspruch 5, dadurch gekennzeichnet, daß das Gummiband aus Kautschuk besteht, insbesondere aus Naturkautschuk.

7. Nässeschutzhülle nach einem der Ansprüche 1 bis 4, dadurch gekennzeichnet, daß an der Hüllenöffnung ein Spanngurt angeordnet ist.

8. Nässeschutzhülle nach Ansprüche 7, dadurch gekennzeichnet, daß die Folie an der Öffnung Schlaufen aufweist.

9. Nässeschutzhülle nach Anspruch 7, dadurch gekennzeichnet, daß an der Öffnung ein Spanngurt mit Klebepunkten oder Nähten befestigt ist.

10. Nässeschutz-Sortiment aus Nässeschutzhüllen nach einem der Ansprüche 1 bis 9, dadurch gekennzeichnet, daß einheitliche Größen für ähnliche Durchmesser von Armen und Beinen zu Verwendung als Bein- und als Arm-Nässeschutz vorgesehen sind.

11. Sortiment nach Anspruch 10, dadurch gekennzeichnet, daß sich die unterschiedlichen Größen im Durchmesser der Hüllenöffnung 2 - 4 cm unterscheiden.

12. Hülle aus physiologisch verträglichem Verbundmaterial, dadurch gekennzeichnet, daß die Hülle an einer Hüllenöffnung einen Bereich aufweist, der beim Zusammenraffen keine wasserdurchlässigen Kanäle bildet.

13. Hülle nach Anspruch 11 oder 12, dadurch gekennzeichnet, daß die Hülle einen 10 bis 30µm dicken mikrogeprägten LLDPE-Streifen aufweist der mittels Schweißnähten auf einer LD-PE Folie befestigt ist.

14. Hülle nach Anspruch 11 oder 13, dadurch gekennzeichnet, daß ein Befestigungsmittel insbesondere ein Gummiband zwischen den Schweißnähten vom LLDPE-Streifen der einem darunter befindlichen Saum 3 eingeschlossen ist.

15. Verfahren zum Abdichten von Körperextremitäten, dadurch gekennzeichnet, daß eine sackförmige Hülle über eine Extremität gezogen wird, und mit einge - schweißtem Gummiband 5 oder einem auf der Hüllenaußenseite aufgebrachten Verschlußstreifen 6 auf der Haut abgedichtet wird, wobei wasserdurchlässige Kanäle vermieden werden.

16. Verfahren zur Herstellung einer Nässeschutzhülle, dadurch gekennzeichnet, daß eine mikrogeprägte kalandrierte Folie 1 zu einer sackförmigen Hülle geschweißt wird und ein Gummiband eingesäumt wird, indem der Hüllenrand umgeschlagen und sein Abschluß an die Hülle geschweißt wird.

17. Verwendung einer wasserdichten Hülle mit Gummiband oder Spanngurt, insbesondere nach einem der vorherigen Ansprüche als Hygieneartikel oder Medizinprodukt, insbesondere als selbstapplizierbarer Nässeschutz.

18. Verwendung der Wasserdichten Hülle nach einem der Ansprüche 1 bis 15 als Kleiderschutz gegen auf Körperextremitäten aufgetragenen Salben, Kräuter, Sole und sonstige Bäder oder Mittel.
